# EUROPÄISCHE PATENTSCHRIFT

(11) **EP 2 406 349 B1**
(45) Veröffentlichungstag und Bekanntmachung des Hinweises auf die Patenterteilung: **18.09.2019**
(21) Anmeldenummer: 10714566.6
(22) Anmeldetag: 18.02.2010
(51) Int. Cl.: C10G 7/08, C10G 45/58, C10G 47/02, C10G 47/22, B01D 3/14, C07C 4/18, C07C 15/06

(54) **VERFAHREN ZUR GEWINNUNG VON REINAROMATEN AUS AROMATENHALTIGEN KOHLENWASSERSTOFFFRAKTIONEN**
METHOD FOR OBTAINING PURE AROMATIC COMPOUNDS FROM HYDROCARBON FRACTIONS CONTAINING AROMATIC COMPOUNDS
PROCÉDÉ DE PRÉPARATION DE COMPOSÉS AROMATIQUES PURS A PARTIR DE FRACTIONS D'HYDROCARBURE RENFERMANT DES COMPOSÉS AROMATIQUES

(30) Priorität: 11.03.2009 DE 102009012265
(43) Veröffentlichungstag der Anmeldung: 18.01.2012
(73) Patentinhaber: thyssenkrupp Industrial Solutions AG, 45143 Essen (DE)
(72) Erfinder: DIEHL, Thomas, 65929 Frankfurt (DE); GEHRKE, Helmut, 59192 Bergkamen (DE); KOLBE, Bärbel, 58452 Witten (DE); WILKEN, Dieter, 59320 Enningerloh (DE)
(74) Vertreter: Brosch, Oliver
(86) Internationale Anmeldenummer: PCT/EP2010/001024
(87) Internationale Veröffentlichungsnummer: WO 2010/102712

(56) Entgegenhaltungen:
- DE-A1- 19 849 651
- GB-A- 1 174 279
- US-A- 3 304 340
- US-A- 3 862 254
- LEI ZHIGANG ET AL: "Extractive distillation: A review", SEPARATION AND PURIFICATION REVIEWS, TAYLOR & FRANCIS INC., PHILADELPHIA, PA, US, vol. 32, no. 2, 1 January 2003 (2003-01-01), pages 121-213, XP009097460, ISSN: 1542-2119, DOI: 10.1081/SPM-120026627

## Beschreibung

Die Erfindung betrifft ein Verfahren zur Gewinnung von Reinaromaten aus aromatenhaltigen Kohlenwasserstofffraktionen und insbesondere aus Reformatbenzin, aus vollhydriertem Pyrolysebenzin, aus druckraffiniertem Kokereibenzol oder aus Gemischen daraus, wobei die extraktive Destillation und die Rückgewinnung des extrahierenden Lösungsmittels in einer einzigen Kolonne stattfinden. Durch das erfindungsgemäße Verfahren werden Aromaten und insbesondere Benzol bereitgestellt, wobei die alkylierten Benzolderivate der Aromatenfraktionen durch hydrierende Dealkylierung in Benzol oder niedere alkylierte Benzolderivate umgewandelt werden. Je nach Auslegung des Verfahrens können diese Produkte in großer Reinheit hergestellt werden. Durch eine neuartige Verfahrensanordnung ist es möglich, gegenüber den Verfahren nach dem Stand der Technik eine deutliche Einsparung an teurem Wasserstoff, der für die hydrierende Dealkylierung eingesetzt wird, und an anlagentechnischem Aufwand zu erreichen. Die Erfindung betrifft auch eine Vorrichtung, mit der dieses Verfahren ausgeführt werden kann.

Aromaten, insbesondere Benzol, Toluol, und Xylole sind wichtige Ausgangsstoffe für die chemische Industrie, vor allem für die Herstellung von Kunststoffen und Chemiefasern. Außerdem werden Aromaten als Oktanzahl-Verstärker im Ottokraftstoff eingesetzt. Für den Einsatz in der chemischen Industrie ist es günstig, wenn die benötigten Aromaten in Form von reinem Benzol bereitgestellt werden, da insbesondere für diese Chemikalie ein großer Bedarf besteht.

Zur Gewinnung von Benzol durch hydrierende Dealkylierung kann man von Reformatbenzin, vollhydriertem Pyrolysebenzin, druckraffiniertem Kokereirohbenzol oder Mischungen daraus als aromatenreiche Kohlenwasserstoffgemische ausgehen. Im folgenden werden diese Einsatzprodukte oder Mischungen daraus als Benzinfraktionen bezeichnet. Die eingesetzten Benzinfraktionen enthalten größere Mengen an Benzolderivaten, insbesondere an Alkylaromaten. Diese können durch thermische oder katalytische Hydrierung in Benzol umgewandelt werden, bei der die Alkylsubstituenten des Benzols durch Umsetzung mit dem Wasserstoff abgespalten und vollständig hydriert werden. Durch dieses Verfahren der hydrierenden Dealkylierung ("HDA") erhält man das erwünschte Benzol. Neben dem erwünschten Benzol erhält man die Alkylsubstituenten in Form von Alkanen.

Beim Reformatbenzin handelt es sich um ein aromatenreiches Benzin, das durch Reformieren und insbesondere katalytisches Reformieren von Naphtha hergestellt wird. Beim Reformieren finden an den in dem Erdöl bzw. Rohöl enthaltenen Alkanen und Cycloalkanen Isomerisierungen, Umlagerungen, Zyklisierungen, Dehydrierungen und ähnliche Reaktionen statt. Das beim katalytischen Reformieren erzeugte aromatenreiche Reformatbenzin stellt ein wichtiges Ausgangsmaterial für die Gewinnung von Aromaten dar.

Beim vollhydrierten Pyrolysebenzin handelt es sich um ein aromatenreiches Benzin, welches beim Dampfspalten von Kohlenwasserstoffen entsteht. Das Dampfspalten von Kohlenwasserstoffen dient in erster Linie zur Erzeugung von niederen Olefinen, insbesondere Ethen. Je nach Siedebereich der für das Dampfspalten eingesetzten Kohlenwasserstoffmischung entsteht eine große Menge eines aromatenreichen Nebenproduktes, das so genannte Pyrolysebenzin, welches für eine Weiterverarbeitung noch durch verschiedene Hydrierschritte (Selektivhydrierung, Vollhydrierung) von ungesättigten Verbindungen und Heteroatomen (Schwefel, Stickstoff, Sauerstoff) befreit wird. Als Produkt der Hydrierschritte enthält man dann eine aromatenreiche Fraktion, die als vollhydriertes Pyrolysebenzin bezeichnet wird.

Beim druckraffiniertem Kokereibenzol handelt es sich ebenfalls um ein aromatenreiches Produkt. Kokereirohbenzol entsteht bei der Verkokung von Kohle. Dieses Kokereirohbenzol enthält, ähnlich wie das Pyrolysebenzin, neben Aromaten noch ungesättigte Verbindungen und heteroatomtragende Verbindungen. Ähnlich der Aufbereitung des Pyrolysebenzins wird auch das Kokereirohbenzol hydrierend behandelt, um sowohl die ungesättigten Verbindungen als auch die heteroatomtragenden Verbindungen umzusetzen. Als Endprodukt dieser Umsetzung erhält man unter anderem ein aromatenreiches Produkt, welches frei von ungesättigten Verbindungen und heteroatomtragenden Verbindungen ist und einer weiteren Verwendung zugeführt werden kann. Dieses aromatenreiche Produkt wird auch als druckraffiniertes Kokereibenzol bezeichnet.

Die eingesetzten aromatenreichen Kohlenwasserstoffgemische können größere Mengen an nichtaromatischen Verbindungen, wie insbesondere Paraffine und Naphthene oder Olefine enthalten, die bei der hydrierenden Dealkylierung ebenfalls hydriert werden, wobei man Alkane als Produkte der Hydrierung erhält. Bei fortschreitender Hydrierung erhält man aus den Alkanen durch Spaltung Methan. Je nach eingesetzter stöchiometrischer Wasserstoffmenge lassen sich bei der hydrierenden Dealkylierung auch niederalkylierte Benzolderivate erhalten, die nicht vollständig zu Benzol dealkyliert wurden.

Die Anwesenheit großer Mengen an nicht-aromatischen Verbindungen im Einsatzstrom zur hydrierenden Dealkylierung erfordert eine entsprechend ausreichende Menge an Wasserstoff für die Durchführung der Reaktion, da Wasserstoff nicht nur benötigt wird, um die Alkylaromaten hydrierend zum Benzol oder zu niederalkylierten Benzolderivaten zu dealkylieren, sondern zusätzlich Wasserstoff für den Abbau der längerkettigen nichtaromatischen Verbindungen hin zu kurzkettigen nichtaromatischen Verbindungen verbraucht wird. Im Falle des vollständigen Abbaus der längerkettigen Verbindungen gelangt man zum Methan. Deshalb ist es eine Aufgabe dieser Erfindung, ein Verfahren zur hydrierenden Dealkylierung bereitzustellen, bei der der Einsatz von Wasserstoff minimiert wird.

Bei der hydrierenden Dealkylierung entsteht neben dem gewünschten Benzol auch ein Gemisch aus kurzkettigen Paraffinen bzw. Methan. Die erzeugte Gasmenge, die durch die Dealkylierung der Aromaten entsteht, kann durch das erfindungsgemäße Verfahren nicht beeinflusst werden. Hingegen ist es auch Aufgabe dieser Erfindung, die Gasmenge zu reduzieren, die durch die Hydrierung der ebenfalls im Eingangsstrom vorhandenen nichtaromatischen Verbindungen entsteht.

Um diese Ziele zu erreichen, ist es günstig, die für die hydrierenden Dealkylierung vorgesehene Kohlenwasserstofffraktion zuerst durch eine extraktive Destillation zu reinigen. Durch die Ausführung der extraktiven Destillation kann ein großer Teil der nicht-aromatischen Verbindungen bereits abgetrennt werden. Das erhaltene Aromatenkonzentrat aus der extraktiven Destillation kann dann einer hydrierenden Dealkylierung zugeführt werden. Da für die kettenverkürzende Hydrierung der nicht-aromatischen Verbindungen, die durch eine extraktive Destillation aus dem Ausgangsgemisch bereits entfernt wurden, kein Wasserstoff verbraucht wird, verringert sich der Wasserstoffverbrauch für die hydrierende Dealkylierung. Außerdem enthält das Stoffgemisch durch Vorschaltung der extraktiven Destillation nach der hydrierenden Alkylierung geringere Anteile an gasförmigen Paraffinen. Dadurch kann das gesamte Verfahren für einen geringeren Gasdurchsatz ausgelegt werden, was sich günstig auswirkt.

Die EP 792928 B1 beschreibt einen Prozess zur Gewinnung von Reinaromaten aus Reformatbenzin. Die Lehre offenbart ein Verfahren, in dem in einer ersten Verfahrensstufe aus dem Reformatbenzin durch fraktionierende Destillation ein Reformatschnitt mit Aromaten einer ausgewählten Kohlenstoffzahl oder mit Aromaten mehrerer ausgewählter Kohlenstoffzahlen Cₓ, C_{y} gewonnen wird, und der Aromatenschnitt in einer zweiten Verfahrensstufe selektiv über einem Hydrierkatalysator hydriert wird, und anschließend in einer dritten Verfahrensstufe die selektiv hydrierten und aromatenhaltigen Produkte aus der zweiten Verfahrensstufe durch Extraktivdestillation und/oder Flüssig-Flüssig-Extraktion in Aromaten und Nichtaromaten aufgetrennt werden. Die Hydrierungsbedingungen in der zweiten Verfahrensstufe werden so eingestellt, dass nichtaromatische ungesättigte Kohlenwasserstoffe, worunter insbesondere Olefine, Diolefine und Triolefine fallen, mithydriert und konjugierte Diolefine und Triolefine möglichst vollständig hydriert werden.

Die DE 1568940 A1 beschreibt ein Verfahren zur Abtrennung von Aromaten aus Kohlenwasserstoffgemischen beliebigen Aromatengehaltes durch Extraktivdestillation, die als nichtaromatische Bestandteile Paraffine, Cycloparaffine, Olefine, Diolefine und organische Schwefelverbindungen enthalten können. Für die Extraktivdestillation werden insbesondere N-substituierte Morpholine verwendet, deren Substituenten nicht mehr als sieben C-Atome aufweisen. Die erhaltene Aromatenfraktion kann einer Nachbehandlung unterzogen werden, wobei eine nachgeschaltete Schwefelsäurewäsche oder eine Bleicherdebehandlung genannt werden. Die Verunreinigungen im Extraktionsmittel, die insbesondere aus ungesättigten Kohlenwasserstoffen bestehen, sammeln sich in der Sumpfphase der Kolonne an und können durch Sumpfphasenhydrierung und Extraktivdestillation von diesem abgetrennt werden.

Die DE 10019196 C1 beschreibt ein Verfahren zur Gewinnung eines aus Benzol und Toluol oder Toluol und Xylol bestehenden Aromatenproduktes hoher Reinheit aus einem Nichtaromaten enthaltenden eng- oder azeotropsiedenden Vorprodukt und eine Vorrichtung zur Durchführung des Verfahrens. Die Extraktivdestillation von Nichtaromaten und die Rückgewinnung des Extraktionsmittels werden in einer einzigen Kolonne durchgeführt, die einen aus zwei parallelen Kammern bestehenden Kolonnenhauptabschnitt, einen Verstärkungsteil oberhalb des Kolonnenhauptabschnittes, einen Abtriebsteil unterhalb des Kolonnenhauptabschnittes und einen Sumpf mit zugeordneter Sumpfbeheizung aufweist. Das Ausgangsbenzin wird zuvor destillativ in mindestens zwei Fraktionen aufgetrennt, von denen eine eine höher siedende Nichtaromaten enthaltende Aromatenfraktion ist, und eine zweite eine niedriger siedende Nichtaromaten enthaltende Aromatenfraktion ist. Die beiden Fraktionen werden an separaten Aufgabestellen der unterseitig und oberseitig offenen Kammer des Kolonnenhauptabschnittes zugeführt, wobei die höher siedende Aromatenfraktion über der niedriger siedenden Aromatenfraktion zugeführt wird. GB 1 1747 279 beschreibt ein Verfahren zur Dealkylierung.

Es besteht deshalb die Aufgabe, ein Verfahren zur Verfügung zu stellen, das ein aromatenreiches Ausgangsbenzin zunächst von den nichtaromatischen Kohlenwasserstoffen durch extraktive Destillation abreichert und das erhaltene Aromatenkonzentrat durch Hydrierung dealkyliert und in Benzol oder dealkylierte Aromaten als Produkt umsetzt. Das entstandene Aromatenkonzentrat soll nach der hydrierenden Dealkylierung rein genug sein, um durch eine einfache Aufarbeitung, wie "Flashen", Rektifikation oder beidem gereinigt werden zu können. Je nach Auslegung soll es auch möglich sein, zwischen dem Prozessschritt der extraktiven Destillation und der hydrierenden Dealkylierung eine weitere Hydrierung durchzuführen, um die restlichen Olefine und mehrfach ungesättigten nichtaromatischen Kohlenwasserstoffe zu entfernen.

Geeignete Einsatzbenzine für die extraktive Destillation die einen genügend hohen Aromatengehalt aufweisen, sind beispielsweise druckraffiniertes Kokereibenzol, vollhydriertes Pyrolysebenzin oder Reformatbenzin, welches in Raffinerien üblicherweise in großen Mengen anfällt. Je nach Ausgangsbenzin kann man dieses auch vor dem Einsatz fraktionierend destillieren, um den Aromatengehalt wie erwünscht zu steigern.

Die Erfindung löst diese Aufgabe durch ein Verfahren, welches von einem aromatenreichen Ausgangsbenzin ausgeht und dieses nacheinander zunächst durch extraktive Destillation reinigt und schließlich durch Hydrierung dealkyliert, und die extraktive Destillation und die Rückgewinnung des extrahierenden Lösungsmittels in einer einzigen Kolonne durchführt. Je nach eingesetzter Wasserstoffmenge und eingesetztem Benzinschnitt erhält man dabei Benzol, oder auch alkylierte Aromaten. Dabei erhält man aus einem aromatenreichen Ausgangsbenzin durch die extraktive Destillation ein an Benzol oder an alkylierten Benzolderivaten reiches Aromatenkonzentrat, das in Abhängigkeit vom eingesetzten Ausgangsgemisch im Wesentlichen Toluole, Xylole und Mesitylene enthält. Die Alkylseitenketten werden bei der Hydrierung in Alkane überführt. Höhere Alkane, die durch die hydrierende Dealkylierung der Seitenketten entstehen, können durch die Hydrierung vollständig in Methan überführt werden. Die erhaltenen Aromatenfraktionen können beispielsweise durch Rektifikation gereinigt werden. Je nach Ausführung der Hydrierung und der Reinigungsschritte kann das Aromatenkonzentrat in jeder gewünschten Reinheit und mit jedem erwünschten Dealkylierungsgrad erhalten werden.

Durch die Durchführung der hydrierenden Dealkylierung nach der extraktiven Destillation wird eine erhebliche Menge an Wasserstoff eingespart, weil die nicht-aromatischen Verbindungen, die in dem Ausgangsbenzin enthalten sind, vor der hydrierenden Dealkylierung durch die extraktive Destillation abgetrennt werden. Die dadurch im Vergleich zu den herkömmlichen Verfahren verringerte Gasmenge an Wasserstoff und auch an Alkanen, die bei der Hydrierung der Olefine entstehen, erlaubt gleichfalls einen höheren Mengendurchsatz an Aromatenprodukt.

Beansprucht wird insbesondere ein Verfahren zur Dealkylierung von einem an C₇- bis C₉-Aromaten reichen Kohlenwasserstoffgemisch, wobei
- das aromatenreiche Ausgangskohlenwasserstoffgemisch zunächst einer extraktiven Destillation unterworfen wird, in der das aromatenhaltige Kohlenwasserstoffgemisch mit einem geeigneten extrahierenden Lösungsmittel destilliert und von diesem befreit wird, wobei man einen an aromatischen Kohlenwasserstoffen stark abgereicherten und lösungsmittelfreien Produktstrom und ein an aromatischen Kohlenwasserstoffen stark angereichertes und lösungsmittelfreies Aromatenkonzentrat erhält, und
- das lösungsmittelfreie Aromatenkonzentrat nach der extrahierenden Destillation in einen Reaktor zur hydrierenden Dealkylierung geführt wird, in dem es mit Wasserstoff zur Reaktion gebracht wird, wobei ein dealkylierter Aromatenstrom als Hauptprodukt und ein paraffinischer Kohlenwasserstoffstrom und ein Methanstrom als Nebenprodukte gebildet werden, und
- der aromatische Kohlenwasserstoffstrom in eine Aufbereitungseinheit geführt wird, und
- die extraktive Destillation und die Rückgewinnung des extrahierenden Lösungsmittels in einer einzigen Kolonne stattfinden.

Das erfindungsgemäße Verfahren kann dazu genutzt werden, Aromaten im erwünschten Alkylierungsgrad in Reinform bereitzustellen. Zur Ausführung des Verfahrens ist es auch möglich, zwischen dem Prozessschritt der extraktiven Destillation und der hydrierenden Dealkylierung eine weitere Hydrierung zur Hydrierung der nichtaromatischen ungesättigen Kohlenwasserstoffe durchzuführen. Dies ist insbesondere dann erforderlich, wenn der Anteil an nichtaromatischen ungesättigten Verbindungen nach der extraktiven Destillation noch unerwünscht hoch liegt. In der Regel ist dieser Zwischenschritt aber nicht erforderlich.

Zur Durchführung des Verfahrens kann jede Vorrichtung genutzt werden, die eine hydrierende Dealkylierung nach der extraktiven Destillation ermöglicht. Zur Durchführung der extraktiven Destillation kommt eine 1-Kolonnenverschaltung zur Anwendung, wie sie allgemein bekannt ist und zum Stand der Technik gehört. Dies ist eine spezielle Ausführung der extraktiven Destillation, die eine Extraktivdestillation und eine Rückgewinnung des extrahierenden Lösungsmittels in einer Kolonne ermöglicht. Dies ist platzsparend und kann reduzierte Investitions- und Betriebskosten ermöglichen.

Zum Einsatz in dem erfindungsgemäßen Verfahren kommen insbesondere Benzinfraktionen, deren Aromatengehalt über 20 Gewichtsprozent liegt. Bevorzugt werden Benzine eingesetzt, deren Aromatengehalt bei über 50 Gewichtsprozent liegt. Ganz besonders bevorzugt werden Benzine eingesetzt, deren Aromatenanteil bei über 80 Gewichtsprozent liegt. Je nach Auslegung kann der Aromatengehalt des Ausgangsbenzines auch durch Vorbehandlungsschritte (z.B. eine Vordestillation) gesteigert werden.

Geeignete Benzine, die für das erfindungsgemäße Verfahren infrage kommen, sind beispielsweise vollhydrierte Pyrolysebenzine. Diese fallen als Crackprodukte bei der Herstellung von Ethen an und besitzen einen hohen Gehalt an Aromaten. Weitere mögliche Einsatzbenzine sind katalytisches Reformatbenzin oder druckraffiniertes Kokereibenzol. Dies kann beispielhaft ein C₇-Schnitt, ein C_{7,8}-Schnitt, ein C_{8,9}-Schnitt oder ein C₇₋₉-Schnitt dieser Benzine sein. Diese Benzine enthalten besitzen aufgrund des Herstellungsprozesses meist einen genügend hohen Aromatenanteil. Druckraffiniertes Kokereibenzol ist insbesondere für die Gewinnung von Reinbenzol geeignet, da dessen Benzolanteil hoch ist. Vor dem Einsatz des Kokereibenzols können Waschprozesse oder Extraktionsprozesse zum Einsatz kommen, um die Benzinfraktion für die Benzolherstellung zu reinigen.

Das Verfahren kann so gestaltet sein, dass man direkt von einem aromatenhaltigen Kohlenwasserstoffgemisch ausgeht, welches in die extraktive Destillation gegeben wird. Das Verfahren kann aber auch so gestaltet werden, dass man die Benzinfraktion zunächst vorbehandelt. Mögliche Vorbehandlungsschritte sind beispielsweise eine Rektifikation, mit der ein Aromatenschnitt des gewünschten Siedepunktbereiches erhalten wird. Möglich sind aber auch Extraktionsschritte oder Waschschritte, wenn das Benzin beispielsweise Verunreinigungen enthält, oder größere Anteile an Paraffinen. Dies kann beispielsweise eine Extraktion mit einem geeigneten Lösungsmittel, mit Wasser oder mit einer Säure sein. Beispielhaft ist dies ein Waschprozess mit Schwefelsäure zur Entfernung der Olefine. Auch eine vorhergehende Extraktivdestillation ist möglich, um ein aromatenarmes Ausgangsbenzin mit Aromaten anzureichern.

Es ist beispielhaft möglich, zur Entfernung von Schwefelverbindungen oder Stickstoffverbindungen einen Oxidationsschritt durchzuführen. Es ist beispielhaft auch möglich, zur Entfernung von Schwefelverbindungen einen zusätzlichen Hydrierschritt durchzuführen. Ist der Gehalt an Olefinen oder Diolefinen hoch, so kann auch eine Vorhydrierung des aromatenhaltigen Ausgangsgemisches durchgeführt werden. Diese Vorhydrierung ist dann Teil des erfindungsgemäßen Verfahrens. Die Vorhydrierung kann auch zur Entschwefelung eingesetzt werden.

Zur Durchführung der extraktiven Destillation kann jedes Lösungsmittel eingesetzt werden, das die Abtrennung von aromatischen Kohlenwasserstoffen aus einer Benzinfraktion ermöglicht. Geeignet hierzu sind insbesondere N-substituierte Lösungsmittel, die 1 bis 8 Kohlenstoffatome im Substituenten enthalten. Besonders geeignet zur extraktiven Destillation ist N-Formylmorpholin. Geeignet zur extraktiven Destillation sind aber auch beispielhaft N-Methylpyrrolidon, Sulfolan, Methylsulfolane, Dimethylsulfoxid (DMSO), Alkylenglykole oder alkylierte Alkylenglykole. Selbstverständlich können auch Lösungsmittelgemische zum Einsatz kommen. Das extraktive Lösungsmittel kann dem Verfahren in seiner Zusammensetzung angepaßt werden. Dem extrahierenden Lösungsmittel kann bei Bedarf auch Wasser zugesetzt werden. Typische Kolonnentemperaturen und -drücke der extraktiven Destillation liegen bei 50 bis 250 °C und 0,2 bis 5 bar.

Aus der extraktiven Destillation erhält man eine an Aromaten abgereicherte Fraktion und das gewünschte Aromatenkonzentrat. Das extrahierende Lösungsmittel wird im Kreis geführt und je nach Bedarf gereinigt oder ergänzt. Das Aromatenkonzentrat wird dann in die Prozessstufe zur Hydrodealkylierung geführt, die sich in der Regel an die extraktive Destillation anschließt. Für einige Ausführungsformen des erfindungsgemäßen Verfahrens kann es vorteilhaft sein, zwischen der extraktiven Destillation und der hydrierenden Dealkylierung eine Vorhydrierung durchzuführen. Die Wahl dieser Verfahrensvariante bleibt dem ausführenden Fachmann überlassen und ist ausdrücklich Teil des erfindungsgemäßen Verfahrens. Für die Prozessstufe der hydrierenden Dealkylierung kommen sowohl thermische als auch katalytische Prozesse in Betracht. Bevorzugt werden jedoch aufgrund der besseren Wirtschaftlichkeit thermische Prozesse.

Ein Verfahren zur Herstellung aromatischer Verbindungen durch thermische Hydrodealkylierung beschreibt die DE 1668719 A1. Ein Gemisch von verdampftem Toluol und Wasserstoff wird bei einer Temperatur von 760 °C innerhalb von 5,4 Sekunden durch eine füllkörperfreie Umsetzungsschlange geführt, die in einem beheizten Erhitzer untergebracht ist. Die Reaktionsführung wird dadurch gesteuert, dass die Temperatur während der Reaktion in engen Grenzen gehalten wird. Der Ablauf aus der Dealkylierungszone wird abgezogen und als Flüssigkeits-Gas-Gemisch in einen Hochdruckabscheider überführt, aus dem die flüssigen Anteile abgezogen werden. Diese können dann beispielhaft durch eine Destillation aufgearbeitet werden. Durch die genannte Reaktion erhält man eine Prozessausbeute für die hydrierende Dealkylierung von 88,5 Prozent Benzol.

Ein Verfahren zur Herstellung aromatischer Verbindungen durch katalytische Hydrodealkylierung beschreibt die US 3197523 A. Die katalytische Hydrodealkylierung wird in einer Dealkylierungszone durchgeführt, die mit einem Katalysator beschickt ist, wobei am Ausgang des Reaktors ein Reaktorausfluss entnommen wird, der in einen Hochdruckabscheider geleitet wird. Dabei werden eine wasserstoffreiche Gasfraktion und eine flüssige Kohlenwasserstofffraktion erhalten, wobei letztere in einen Niederdruckabscheider geführt wird, wo leichtere Kohlenwasserstoffe abgetrennt werden und eine weitere Kohlenwasserstofffraktion erhalten wird, die destilliert wird, wobei man die gewünschte Benzolfraktion erhält. Typische Drücke in der Dealkylierungszone betragen 20 bis 100 bar, typische Temperaturen in der Dealkylierungszone betragen 540 bis 800 °C.

In einer günstigen großtechnischen Ausführungsform wird das flüssige Ausgangsgemisch mit vorerhitztem Wasserstoff und einem wasserstoffreichen Recyclegas verdampft und in einen Vorbehandlungsreaktor gegeben, in der die in dem Gemisch enthaltenen restlichen Diolefine und Triolefine hydriert werden, und die in dem Gemisch enthaltenen Schwefelverbindungen, insbesondere Thiophen, zu H₂S hydriert werden. Das Reaktionsgemisch wird dann durch eine Befeuerung erhitzt und durch den eigentlichen Dealkylierungsreaktor geleitet. Der erhaltene Reaktorausfluss wird von dem wasserstoffreichen Ausgangsgas getrennt, die erhaltene flüssige Fraktion entspannt, je nach Anforderung teilweise in den Reaktor zurückgeführt und das aromatische Produkt destilliert. Der Reaktorausfluss wird zur Erzeugung von Dampf und Wärme genutzt. Je nach Reinheit werden das erhaltene Reinbenzol oder die dealkylierten Aromaten durch Festbetten mit Tonkörpern geleitet. Je nach Reinheit kann auch die Vorbehandlungsstufe ausgelassen werden.

An die Prozessstufe der hydrierenden Dealkylierung schließt sich ein Verfahrensschritt der Aufbereitung an. Je nach Reinheit des erhaltenen Produktes kann eine sogenannte "Flash"-Destillation verwendet werden, um den apparativen Aufwand gering zu halten. Es kann aber auch beispielhaft eine Destillation oder eine Ausfrierstufe erfolgen.

Bevorzugt wird mit dem erfindungsgemäßen Verfahren Reinbenzol gewonnen. Je nach Ausgangsbenzin oder Dealkylierungsgrad können aber auch beispielhaft C₇-Aromaten oder ein C_{7/8}-Schnitt erhalten werden.

Wegen der günstigeren Prozessbedingungen und wegen der höheren Prozessausbeuten wird in der Regel die thermische Dealkylierung bevorzugt, da diese einfacher durchzuführen ist und bei richtiger Prozessführung hohe Prozessausbeuten erbringt. Dadurch kommt die Einsparung an Wasserstoff, die man durch eine Durchführung der Hydrodealkylierung nach der extraktiven Destillation gewinnt, zum Tragen. Bei dieser Verfahrensvariante läßt sich außerdem ein höherer Produktdurchsatz erreichen, da durch den verringerten Anteil an nichtaromatischen Verbindungen nach der extraktiven Destillation weniger gasförmige Nebenprodukte entstehen. Dadurch wird die Menge insbesondere an niedrigsiedenden Kohlenwasserstoffen im Produkt verringert, was einen niedrigeren Gasballast im Prozess und damit eine verbesserte Durchführbarkeit und Wirtschaftlichkeit des Prozesses zur Folge hat.

Beansprucht wird auch eine Vorrichtung, mit der das erfindungsgemäße Verfahren ausgeführt wird. Beansprucht wird insbesondere eine Vorrichtung zur Dealkylierung eines an Aromaten reichen Kohlenwasserstoffgemisches, umfassend
- eine Kolonne für die extraktive Destillation, einen Reaktor zur Hydrierung des anfallenden Aromatenkonzentrates, eine Reinigungseinheit zur Aufbereitung des anfallenden aromatischen Produktes, wobei
- die Kolonne für die extraktive Destillation, der Hydrierreaktor und die Reinigungseinheit so angeordnet sind, dass im Prozessfluss nacheinander eine extraktive Destillation von aromatischen Kohlenwasserstoffen aus einem kohlenwasserstoffhaltigen Gemisch, eine Entfernung von extrahierendem Lösungsmittel aus dem aromatischen Produktstrom, eine hydrierende Dealkylierung von Aromaten zum Erhalt eines dealkylierten aromatischen Produktes und eine Aufbereitung des erhaltenen aromatischen Produktes erfolgen, und
- die Vorrichtung eine Kolonne umfasst, die eine extrahierende Destillation und die Rückgewinnung des extrahierenden Lösungsmittels in einer einzigen Kolonne ermöglicht.

Zur Anwendung können dabei beliebige Ausführungen für die genannten Vorrichtungen kommen. Diese können Vorrichtungen enthalten, die für eine Destillation notwendig sind oder diese unterstützen. Dies können beispielsweise Reboiler, Heizvorrichtungen oder Pumpen zur Aufrechterhaltung eines Vakuums sein. Diese Vorrichtungen sind dann ausdrücklich Teil der erfindungsgemäßen Vorrichtung.

Der Verfahrensschritt der extraktiven Destillation wird in einer Kolonne durchgeführt, die die extraktive Destillation und die Trennung des extrahierenden Lösungsmittels in einer Kolonne ermöglicht. Ein Beispiel für eine solche Kolonne, die eine extraktive Destillation und eine Trennung des extrahierenden Lösungsmittels von der aromatenhaltigen Produktfraktion in einer Kolonne ermöglicht, beschreibt die DE 19849651 A1. Das extrahierende Lösungsmittel wird dabei im Kreis geführt. Die Kolonne enthält zur Aufrechterhaltung der Destillationswärme Erhitzungseinrichtungen für das extrahierende Lösungsmittel. Sie kann auch Einrichtungen zum Erhitzen des Aromatenstroms enthalten, wobei beispielhaft Reboiler im Sumpf der Kolonne genannt seien. Die Kolonne, die eine extraktive Destillation in einer Kolonnenvorrichtung ermöglicht, kann jedoch letztlich beliebig geartet sein.

Beansprucht wird insbesondere auch eine Vorrichtung zur Dealkylierung eines an Aromaten reichen Kohlenwasserstoffgemisches, die eine Kolonne umfasst, die eine extraktive Destillation und die Rückgewinnung des extrahierenden Lösungsmittels in einer einzigen Kolonne ermöglicht, wobei diese Kolonne
- einen Abtriebsteil unterhalb des Kolonnenhauptabschnittes, einen Verstärkungsteil oberhalb des Kolonnenhauptabschnittes, einen aus zwei parallelen Kammern bestehenden Kolonnenhauptabschnitt, und einen Kolonnensumpf mit zugeordneter Sumpfheizung aufweist, und
- die eine Kammer des Kolonnenhauptabschnittes oberseitig und unterseitig gegenüber dem Kolonneninnenraum offen ist, und die andere Kammer oberseitig gegenüber dem Kolonneninnenraum geschlossen und unterseitig gegenüber dem Kolonneninnenraum offen ist, wobei alle Kammern Einbauten zur Verbesserung des Stoffaustausches enthalten, und oberhalb der Einbauten der Kammer, die oberseitig gegenüber dem Kolonneninnenraum geschlossen und unterseitig gegenüber dem Kolonneninnenraum offen ist, Einrichtungen für den dampfförmigen Abzug eines extraktionsmittelfreien Produktes sowie für den Rückfluß eines verflüssigten Produktteilstromes vorhanden sind.

Zur Durchführung der hydrierenden Dealkylierung kommen Vorrichtungen in Betracht, mit denen eine hydrierende Dealkylierung nach dem Stand der Technik durchgeführt werden kann, mit denen eine Recyclierung des überschüssigen Hydrierwasserstoffs durchgeführt werden kann und mit denen eine Reinigung des Reinbenzolstroms oder der Reinaromatenfraktion durchgeführt werden kann und mit der gegebenenfalls auch eine Weiterverwendung des anfallenden Methanstroms möglich ist. Hierzu gehören beispielsweise Füllkörperkolonnen, Verdampfer, Destillationstürme, Hydrierreaktoren mit Katalysatorfüllungen oder Diffusionsabscheider. Die Vorrichtung zur Durchführung der hydrierenden Dealkylierung kann letztlich beliebig geartet sein, muss aber zur hydrierenden Dealkylierung eines Aromatenstromes geeignet sein, der bei der extraktiven Destillation eines aromatenhaltigen Kohlenwasserstoffgemisches typischerweise anfällt.

Zur Vorbehandlung des Ausgangsbenzines und des aromatenhaltigen Ausgangsgemisches gehören schliesslich auch Vorrichtungen, die zur Durchführung dieser Verfahrensschritte notwendig sind. Zur Vorbehandlung können beispielhaft Destillationskolonnen zur fraktionierenden Destillation, Extraktionstürme oder Ausfrierungsvorrichtungen erforderlich sein. Diese Vorrichtungen sind bei Verwendung im erfindungsgemäßen Verfahren ausdrücklich Teil des erfindungsgemäßen Verfahrens. Teil des erfindungsgemäßen Verfahrens sind auch Verfahrensschritte zur Reinigung des aromatenhaltigen Produktes nach der hydrierenden Dealkylierung. Hierzu kommen beispielhaft Destillationskolonnen zur fraktionierenden Destillation, "Flash"-Destillationseinheiten, Festbetten mit Tonkörpern oder Ausfrierstufen in Betracht.

Schließlich können alle Vorrichtungsteile Einrichtungen enthalten, die zur Aufrechterhaltung eines Betriebes zur extraktiven Destillation und zur hydrierenden Dealkylierung von Aromaten erforderlich sind. Dies sind beispielsweise Kompressoren, Turbinen, Pumpen, und Ventile. Dies sind selbstverständlich auch Einrichtungen zur Aufrechterhaltung der erforderlichen Betriebstemperaturen, wobei Heizvorrichtungen, Brenner, Wärmetauscher oder Kühlungsvorrichtungen hier beispielhaft genannt seien. Schließlich enthalten diese auch die erforderlichen Regeleinrichtungen, wie Thermostaten oder Steuerungseinrichtungen.

Das erfindungsgemäße Verfahren und die erfindungsgemäße Vorrichtung bieten den Vorteil einer konventionellen Geräteanordnung zur Herstellung von reinen aromatenhaltigen Produkten, für die eine erheblich geringere Menge an Wasserstoff verbraucht wird als bei herkömmlichen Verfahren. Durch dieses Verfahren kann zudem ein erheblich höherer Durchsatz erreicht werden als bei einer konventionellen Anordnung zur Herstellung von Reinaromaten. Die beschriebene Anordnung eignet sich insbesondere zur Herstellung von Reinbenzol, ist aber auch zur Herstellung alkylierter aromatenhaltiger Fraktionen, wie beispielsweise Toluol, geeignet.

Die erfindungsgemäße Ausgestaltung eines Verfahrens zur Erzeugung von aromatenhaltigen Kohlenwasserstoffgemischen wird anhand eines Beispiels erläutert, das die Vorgehensweise und die Zusammensetzung des Ausgangsgemisches und des Aromatenkonzentrates nach der extraktiven Destillation beschreibt.

Beispiel: Ein aromatenhaltiges Kohlenwasserstoffgemisch aus vollhydriertem Pyrolysebenzin mit einem Aromatengehalt von 86 Massenprozent wird einer extraktiven Destillation unterzogen, wobei die in der DE 19849651 A1 genannte Vorrichtung und N-Formylmorpholin als extrahierendes Lösungsmittel zum Einsatz kommen. Die Zusammensetzung des aromatenhaltiges Ausgangsgemisches vor und des Aromatenkonzentrats nach der extraktiven Destillation wird in Tabelle 1 angegeben. Die Zusammensetzungen wurden durch gaschromatographische Analyse der Fraktionen bestimmt. Im Anschluss an die extraktive Destillation wird eine hydrierende Dealkylierung durchgeführt, bei der das aus der extraktiven Destillation erhaltene Gemisch in einem Anlagenteil zur hydrierenden Dealkylierung mit Wasserstoff umgesetzt wird. Dabei wird ein aromatenhaltiges dealkyliertes Produkt erhalten.

Unterwirft man das im Beispiel genannte Ausgangsgemisch einer hydrierenden Dealkylierung, so werden pro 1000 kg Ausgangsgemisch 38,7 kg Wasserstoff benötigt und 718 kg Benzol erhalten. Die Gasbelastung im Reaktoraustritt beträgt (ohne Berücksichtigung eines erforderlichen Wasserstoffüberschusses) 636 Nm³. Unterwirft man hingegen das nach der extraktiven Destillation erhaltene Aromatenkonzentrat einer hydrierenden Dealkylierung, so werden pro 1000 kg Aromatenkonzentrat 24,4 kg Wasserstoff benötigt und 825 kg Benzol erhalten. Die Gasbelastung im Reaktoraustritt beträgt (ohne Berücksichtigung eines erforderlichen Wasserstoffüberschusses) 507 Nm³. Dieses Beispiel demonstriert die vorteilhafte Ausführung der Erfindung.

Die erfindungsgemäße Ausgestaltung eines Prozessflusses zur Erzeugung von dealkylierten aromatenhaltigen Kohlenwasserstoffgemischen wird anhand von drei Zeichnungen genauer erläutert, wobei das erfindungsgemäße Verfahren nicht auf diese Ausführungsformen beschränkt ist.

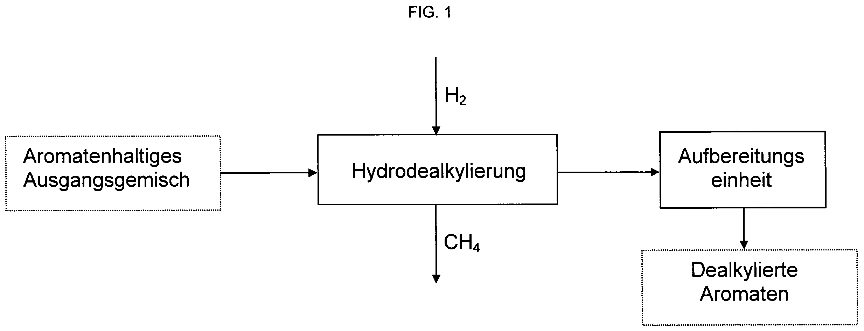

FIG. 1 zeigt ein Prozessflussdiagramm einer hydrierenden Dealkylierung, wie es beispielhaft im Stand der Technik ausgeführt wird. Das aromatenhaltige Ausgangsgemisch wird einer dehydrierenden Alkylierung zugeführt. Zur Ausführung der Reaktion wird dieser Reaktionsstufe weiterhin Wasserstoff zugeführt. Bei der hydrierenden Dealkylierung fällt Methan aus der hydrierenden Abspaltung der Alkylseitenketten an. Man erhält einen Produktstrom an dealkylierten Aromaten, der in einer Aufbereitungseinheit gereinigt wird. Dabei erhält man ein reines Produkt an dealkylierten Aromaten. Olefine, die in dem Aromatenkonzentrat enthalten sind, werden mithydriert.

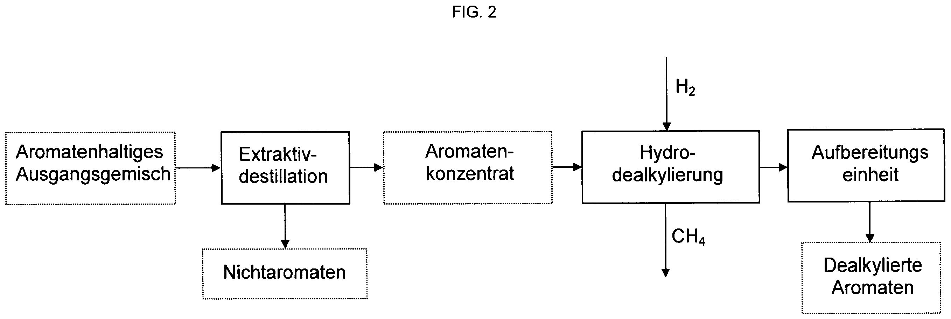

FIG. 2 zeigt ein Prozessflussdiagramm des erfindungsgemäßen Verfahrens. Ein aromatenhaltiges Ausgangsgemisch aus Kohlenwasserstoffen wird in eine extraktive Destillation gegeben. Dabei wird ein an Aromaten abgereicherter Kohlenwasserstoffstrom und ein an Aromaten angereicherter Kohlenwasserstoffstrom erhalten. Der an Aromaten angereicherte Kohlenwasserstoffstrom wird als Aromatenkonzentrat in die Prozesseinheit zur hydrierenden Dealkylierung geführt. Zur Ausführung der Reaktion wird dieser Reaktionsstufe weiterhin Wasserstoff zugeführt. Bei der hydrierenden Dealkylierung fällt Methan aus der hydrierenden Abspaltung der Alkylseitenketten an. Man erhält einen Produktstrom an dealkylierten Aromaten, der in einer Aufbereitungseinheit gereinigt wird. Dabei erhält man ein reines Produkt an dealkylierten Aromaten.

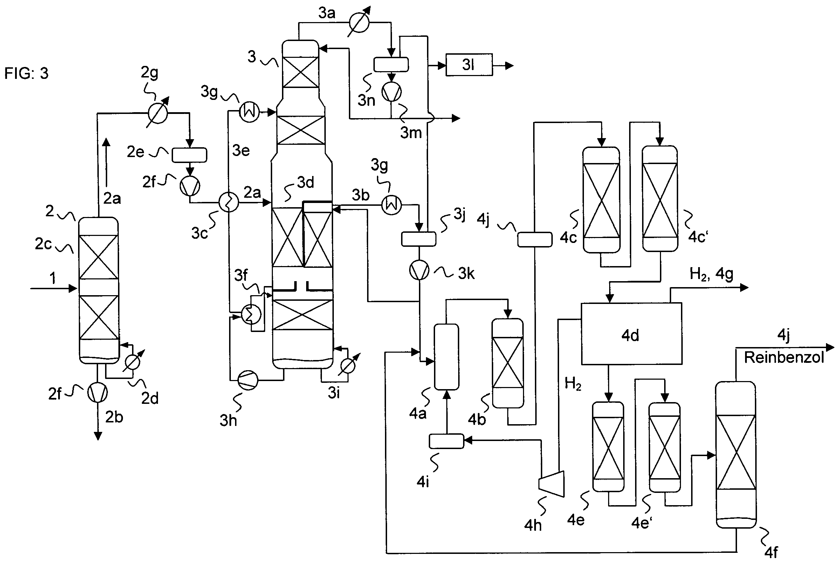

FIG. 3 zeigt eine erfindungsgemäße Vorrichtung zur Ausführung des erfindungsgemäßen Verfahrens. Das aromatenhaltige Ausgangsbenzin (**1**) wird in eine Destillationskolonne (**2**) geführt, in der das Ausgangsbenzin in eine niedriger siedende (**2a**) und eine höher siedende (**2b**), jeweils Nichtaromaten enthaltende Aromatenfraktion aufgetrennt wird. Beispielhaft kann es sich bei diesen Fraktionen um eine vorwiegend benzolhaltige (**2a**) und eine vorwiegend Toluol und Xylole enthaltende Fraktion (**2b**) handeln. Die höher siedende Fraktion (**2b**) kann ausgeschleust werden. Diese Fraktionen enthalten typischerweise auch Olefine. Je nach Zusammensetzung des Ausgangsbenzines können auch zwei Destillationskolonnen verwendet werden. Die Auftrennung erfolgt in einer Vordestillationskolonne (**2**) mit speziellen Vorrichtungen (**2c**), die eine verbesserte Auftrennung des Gemisches ernöglichen. Die Destillation enthält in dieser Zeichnung auch Vorrichtungen (**2d**) zur Aufrechterhaltung der für die Destillation vorgesehenen Temperatur, sogenannte Reboiler. Ausserdem sind noch Zwischenbehälter (**2e**), Kompressoren (**2f**) und Zwischenerhitzer (**2g**) vorhanden. Die beiden Fraktionen werden dann an getrennten Aufgabestellen in die Destillationskolonne (**3**) gegeben, die für die extraktive Destillation vorgesehen ist. Die niedriger siedende Aromatenfraktion (**2a**) wird über Zwischenerhitzer (**2g**), Kompressoren (**2f**) und Wärmetauscher (**3c**) auf ein für die extraktive Destillation vorgesehenes Temperatur- und Druckniveau gebracht. In dieser Kolonne für die extraktive Destillation (**3**) wird das Aromatengemisch in ein aromatenarmes (**3a**) und ein aromatenreiches (**3b**) Kohlenwasserstoffgemisch aufgetrennt, wobei man die aromatenarme Kohlenwasserstofffraktion (**3a**) am Kopf der Kolonne erhält und die aromatenhaltige, nicht-aromaten-arme Kohlenwasserstofffraktion als Aromatenkonzentrat (**3b**) an einem Seitenabzug der Kolonne entnehmen kann. Die Aufgabe des Ausgangsgemisches (**2a**) erfolgt über einer speziellen Destillationsvorrichtung mit zwei Kammern (**3d**), die im Kolonnenhauptabschnitt untergebracht ist und die zur Verbesserung des Stoffaustausches mit Einbauten versehen ist. Die spezielle Gestaltung der Kolonne erlaubt es, das extrahierende Lösungsmittel im Kreis zu führen (**3e**) und nur zeitweise zersetztes oder entlaufenes Lösungsmittel zu ersetzen. Die Kolonne enthält Wärmetauscher (**3c, 3f, 3g**), um das extrahierende Lösungsmittel auf einer für die extraktive Destillation notwendigen Temperatur zu halten. Die Kolonne enthält ferner auch Einrichtungen, die den gesamten Stoffstrom auf einer für die extraktive Destillation notwendigen Druck und Temperatur halten, beispielsweise einen Kompressor (**3h**) und einen Reboiler (**3i**). Die Bereitstellung des Aromatenkonzentrates erfolgt über einen Zwischenbehälter (**3j**) und einen Kompressor (**3k**). Die aromatenarme Kohlenwasserstofffraktion (**3a**) enthält neben den Paraffinen, Olefine, Diolefine und Triolefine und wird über einen Sammelbehälter **(3l)** ausgeschleust. Am Kopf der Kolonne befindet sich eine Vakuumpumpe (**3m**) zur Aufrechterhaltung eines Vakuums und ein Zwischenbehälter (**3n**).

Das Aromatenkonzentrat (**3b**) wird dann in den Anlagenteil zur hydrierenden Dealkylierung geführt. Dieser enthält in der hier dargestellten Ausführungsform einen Verdampfer (**4a**), einen Vorbehandlungsreaktor (**4b**), zwei Dealkylierungsreaktoren (**4c,4c'**), eine Wasserstoffabscheider (**4d**), zwei Nachbehandlungstürme zur Reinigung (**4e,4e'**) und eine Destillationskolonne (**4f**). Das aromatenhaltige Konzentrat wird zunächst in den Verdampfer geführt (**4a**) und gelangt in Dampfform in den Vorbehandlungsreaktor (**4b**). Dort wird ein möglicher Rest an Olefinen oder Diolefinen hydriert. Nach dieser Vorbehandlung wird das aromatenhaltige Gemisch in die Dealkylierungsreaktoren (**4c**,**4c**') geführt, die hier zweifach vorhanden sind. Diese bestehen in der Regel aus durch Brenner befeuerten Röhren, durch die das zu dealkylierende Aromatenkonzentrat geführt wird. Dabei entstehen die dampffömigen Aromaten und Alkane aus den Alkylketten, die unter den angewendeten Bedingungen in der Regel zu Methan umgewandelt werden. Das erhaltene Gasgemisch gelangt in einen Wasserstoffabscheider (**4d**), der den Wasserstoff abtrennt und entweder mit dem Methan ausschleust (**4g**) oder über einen Kompressor (**4h**) und einen Zwischenbehälter (**4i**) in den Verdampfer (**4a**) zurückführt. Das aus dem Wasserstoffabscheider (**4d**) stammende Aromatengemisch wird in Füllkörperkolonnen geleitet (**4e,4e'**), die in einer typischen Ausführung mit Tonkörperfüllkörpern versehen sind. Darin werden beispielsweise Polymerisationsprodukte der Dealkylierungsreaktion abgetrennt. Nach dieser Reinigung erhält man ein Benzol, das sehr rein ist und durch eine Destillation in einer Destillationskolonne (**4f**) entsprechend aufgereinigt werden kann. Das Reinbenzol (**4j**) wird am Kopf der Kolonne entnommen. Je nach den Bedingungen der Dealkylierung kann man auch höher alkylierte Produkte, beispielsweise Toluol oder Xylole, erhalten.

### Bezugszeichenliste

- 1: Ausgangsbenzin
- 2: Destillationskolonne
- 2a: Niedriger siedende Aromatenfraktion
- 2b: Höher siedende Aromatenfraktion
- 2c: Vorrichtungen zur Verbesserung der Trennung
- 2d: Reboiler
- 2e: Zwischenbehälter
- 2f: Kompressor
- 2g: Zwischenerhitzer
- 3: Kolonne zur extraktiven Destillation
- 3a: Olefinreiches Kohlenwasserstoffgemisch
- 3b: Olefinarmes Aromatenkonzentrat
- 3c: Wärmetauscher
- 3d: Zwei Kammern der Extraktionsdestillationskolonne
- 3e: Im Kreis geführtes extrahierendes Lösungsmittel
- 3f, 3g: Wärmetauscher
- 3h: Kompressor
- 3i: Reboiler
- 3j: Zwischenbehälter
- 3k: Kompressor
- 3l: Sammelbehälter
- 3m: Vakuumpumpe
- 3n: Zwischenbehälter
- 4a: Verdampfer
- 4b: Vorbehandlungsreaktor
- 4c,4c': Dealkylierungsreaktoren
- 4d: Wasserstoffabscheider
- 4e,4e': Nachbehandlungstürme
- 4f: Destillationskolonne
- 4g: Methan- und Wasserstoffausschleusung
- 4h: Kompressor
- 4i: Zwischenbehälter
- 4j: Reinbenzolabzug

**Tabelle 1**

| | Aromatenhaltiges Ausgangsgemisch | Aromatenkonzentrat |
|---|---|---|
| Benzol | 2,29 | frei |
| Toluol | 71,67 | 85,07 |
| C₈-Aromaten (Ethylbenzol und Xylole) | 11,79 | 14,14 |
| C₉-Aromaten | 0,20 | 0,37 |
| *n*-Heptan | 1,75 | frei |
| *iso*-Heptane | 0,07 | frei |
| C₇-Naphthene | 5,57 | frei |
| *n*-Octan | 0,88 | frei |
| *iso*-Octane | 2,88 | < 0,01 |
| C₈-Nahthene | 1,49 | < 0,01 |
| *n*-Nonan | 0,05 | 0,11 |
| *iso*-Nonan | 0,44 | 0,08 |
| C₉-Naphhtene | 0,60 | 0,09 |
| *n*-Decan | frei | frei |
| *iso*-Decan | 0,07 | 0,08 |
| C₁₀-Naphthene | frei | frei |
| Andere | Summe zu Hundert | Summe zu Hundert |
| Summe Aromaten | 85,99 | 99,56 |
| Summe (n+i)-Paraffine | 6,01 | 0,15 |
| Summe Naphthene | 7,63 | 0,05 |

## Patentansprüche

1. Verfahren zur Dealkylierung von einem an C₇ - bis C₉-Aromaten reichen Kohlenwasserstoffgemisch, wobei
• das aromatenreiche Ausgangskohlenwasserstoffgemisch zunächst einer extraktiven Destillation unterworfen wird, in der das aromatenhaltige Kohlenwasserstoffgemisch mit einem geeigneten extrahierenden Lösungsmittel destilliert und von diesem befreit wird, wobei man einen an aromatischen Kohlenwasserstoffen stark abgereicherten und lösungsmittelfreien Produktstrom und ein an aromatischen Kohlenwasserstoffen stark angereichertes und lösungsmittelfreies Aromatenkonzentrat erhält,
**dadurch gekennzeichnet, dass**
• das lösungsmittelfreie Aromatenkonzentrat nach der extrahierenden Destillation in einen Reaktor zur hydrierenden Dealkylierung geführt wird, in dem es mit Wasserstoff zur Reaktion gebracht wird, wobei ein dealkylierter Aromatenstrom als Hauptprodukt und ein paraffinischer Kohlenwasserstoffstrom und ein Methanstrom als Nebenprodukte gebildet werden, und
• der aromatische Kohlenwasserstoffstrom in eine Aufbereitungseinheit geführt wird, und
• die extraktive Destillation und die Rückgewinnung des extrahierenden Lösungsmittels in einer einzigen Kolonne stattfinden.

2. Verfahren nach Anspruch 1, **dadurch gekennzeichnet, dass** es sich bei der Aufbereitungseinheit um einefraktionierende Destillation handelt.

3. Verfahren nach Anspruch 1, **dadurch gekennzeichnet, dass** es sich bei der Aufbereitungseinheit um eine "Flash"-Destillationsstufe handelt.

4. Verfahren zur Dealkylierung eines an Aromaten reichen Kohlenwasserstoffgemisches nach einem der Ansprüche 1 bis 3, **dadurch gekennzeichnet, dass** es sich bei dem aromatenhaltigen Ausgangsprodukt um vollhydriertes Pyrolysebenzin handelt.

5. Verfahren zur Dealkylierung eines an Aromaten reichen Kohlenwasserstoffgemisches nach einem der Ansprüche 1 bis 3, **dadurch gekennzeichnet, dass** es sich bei dem aromatenhaltigen Ausgangsprodukt um katalytisches Reformatbenzin handelt.

6. Verfahren zur Dealkylierung eines an Aromaten reichen
Kohlenwasserstoffgemisches nach einem der Ansprüche 1 bis 3, **dadurch gekennzeichnet, dass** es sich bei dem aromatenhaltigen Ausgangsprodukt um druckraffiniertes Kokerei-Benzol handelt.

7. Verfahren zur Dealkylierung eines an Aromaten reichen Kohlenwasserstoffgemisches nach einem der Ansprüche 1 bis 6, **dadurch gekennzeichnet, dass** es sich bei dem dealkylierten und aufbereiteten Aromatenstrom um reines Benzol handelt.

8. Verfahren zur Dealkylierung eines an Aromaten reichen Kohlenwasserstoffgemisches nach einem der Ansprüche 1 bis 7, **dadurch gekennzeichnet, dass** das aromatenhaltige Ausgangsprodukt vor der Zuführung in die extraktive Destillation in einer Destillationskolonne fraktionierend destilliert wird.

9. Verfahren zur Dealkylierung eines an Aromaten reichen Kohlenwasserstoffgemisches nach einem der Ansprüche 1 bis 8, **dadurch gekennzeichnet, dass** das aromatenhaltige Ausgangsprodukt vor der Zuführung in die extraktive Destillation mit Wasser, mit einer Säure, mit einem geeigneten Lösungsmittel oder mit einem Gemisch dieser Stoffe in Kontakt gebracht wird.

10. Verfahren zur Dealkylierung eines an Aromaten reichen Kohlenwasserstoffgemisches nach einem der Ansprüche 1 bis 9, **dadurch gekennzeichnet, dass** das aromatenhaltige Ausgangsprodukt vor der Zuführung in die extraktive Destillation einer Hydrierung unterzogen wird.

11. Verfahren zur Dealkylierung eines an Aromaten reichen
Kohlenwasserstoffgemisches nach einem der Ansprüche 1 bis 9, **dadurch gekennzeichnet, dass** zwischen dem Verfahrensschritt der extraktiven Destillation und der hydrierenden Dealkylierung eine Hydrierung durchgeführt wird.

12. Verfahren zur Dealkylierung eines an Aromaten reichen
Kohlenwasserstoffgemisches nach einem der Ansprüche 1 bis 11, **dadurch gekennzeichnet, dass** es sich bei dem extrahierenden Lösungsmittel um ein N-substituiertes Lösungsmittel mit 1 bis 8 Kohlenstoffatomen im Substituenten handelt.

13. Verfahren zur Dealkylierung eines an Aromaten reichen
Kohlenwasserstoffgemisches nach einem der Ansprüche 1 bis 11, **dadurch gekennzeichnet, dass** es sich beim dem extrahierenden Lösungsmittel um N-Formylmorpholin handelt.

14. Verfahren zur Dealkylierung eines an Aromaten reichen Kohlenwasserstoffgemisches nach einem der Ansprüche 1 bis 11, **dadurch gekennzeichnet, dass** es sich bei dem extrahierenden Lösungsmittel zur extraktiven Destillation um N- Methylpyrrolidon, Sulfolan, Methylsulfolan, um ein Alkylenglykol oder ein alkyliertes Alkylenglykol oder ein Gemisch dieser Stoffe handelt.

15. Verfahren zur Dealkylierung eines an Aromaten reichen
Kohlenwasserstoffgemisches nach einem der Ansprüche 12 bis 14, **dadurch gekennzeichnet, dass** das extrahierende Lösungsmittelgemisch zur extraktiven Destillation Wasser enthält.

16. Verfahren zur Dealkylierung eines an Aromaten reichen Kohlenwasserstoffgemisches nach einem der Ansprüche 1 bis 15, **dadurch gekennzeichnet, dass** es sich bei dem Hydrierungsschritt zur hydrierenden Dealkylierung um einen thermischen Prozess handelt.

17. Verfahren zur Dealkylierung eines an Aromaten reichen
Kohlenwasserstoffgemisches nach einem der Ansprüche 1 bis 15, **dadurch gekennzeichnet, dass** es sich bei dem Hydrierungsschritt zur hydrierenden Dealkylierung um einen katalytischen Prozess handelt.

18. Vorrichtung zur Dealkylierung eines an Aromaten reichen Kohlenwasserstoffgemisches, umfassend
• eine Rektifizierkolonne für die Extraktivdestillation, eine Stripperkolonne für die Entfernung des extrahierenden Lösungsmittels, einen Reaktor zur Hydrierung des anfallenden Aromatenkonzentrates, eine Reinigungseinheit zur Aufbereitung des anfallenden Aromatenkonzentrates,
**dadurch gekennzeichnet, dass**
• die Rektifizierkolonne, die Stripperkolonne, der Hydrierreaktor und die Reinigungseinheit so angeordnet sind, dass im Prozessfluss nacheinander eine extraktive Destillation von aromatischen Kohlenwasserstoffen aus einem kohlenwasserstoffhaltigen Gemisch, eine Entfernung von extrahierendem Lösungsmittel aus dem aromatischen Produktstrom, eine hydrierende Dealkylierung von Aromaten zum Erhalt eines dealkylierten Aromatenkonzentrates und eine Aufbereitung des erhaltenen Aromatenkonzentrates erfolgen, und
• die Vorrichtung eine Kolonne umfasst, die eine extraktive Destillation und die Rückgewinnung des extrahierenden Lösungsmittels in einer einzigen Kolonne ermöglicht.

19. Vorrichtung zur Dealkylierung eines an Aromaten reichen Kohlenwasserstoffgemisches nach Anspruch 18, **dadurch gekennzeichnet, dass** sie eine Kolonne umfasst, die eine extraktive Destillation und die Rückgewinnung des extrahierenden Lösungsmittels in einer einzigen Kolonne ermöglicht, wobei diese Kolonne
• einen Abtriebsteil unterhalb des Kolonnenhauptabschnittes, einen Verstärkungsteil oberhalb des Kolonnenhauptabschnittes, einen aus zwei parallelen Kammern bestehenden Kolonnenhauptabschnitt, und einen Kolonnensumpf mit zugeordneter Sumpfheizung aufweist, und
• die eine Kammer des Kolonnenhauptabschnittes oberseitig und unterseitig gegenüber dem Kolonneninnenraum offen ist, und die andere Kammer oberseitig gegenüber dem Kolonneninnenraum geschlossen und unterseitig gegenüber dem Kolonneninnenraum offen ist, wobei alle Kammern Einbauten zur Verbesserung des Stoffaustausches enthalten, und oberhalb der Einbauten der Kammer, die oberseitig gegenüber dem Kolonneninnenraum geschlossen und unterseitig gegenüber dem Kolonneninnenraum offen ist, Einrichtungen für den dampfförmigen Abzug eines extraktionsmittelfreien Produktes sowie für den Rückfluß eines verflüssigten Produktteilstromes vorhanden sind.

20. Vorrichtung zur Dealkylierung eines an Aromaten reichen Kohlenwasserstoffgemisches nach einem der Ansprüche 18 bis 19, **dadurch gekennzeichnet, dass** diese eine Destillationseinheit zur fraktionierenden Destillation umfasst, die im Prozessfluss hinter der Einheit zur hydrierenden Dealkylierung angeordnet ist.

21. Vorrichtung zur Dealkylierung eines an Aromaten reichen Kohlenwasserstoffgemisches nach einem der Ansprüche 18 bis 19, **dadurch gekennzeichnet, dass** diese eine "Flash"-Destillationsstufe umfasst, die im Prozessfluss hinter der Einheit zur hydrierenden Dealkylierung angeordnet ist.

## Claims

1. Process for dealkylation of hydrocarbon mixture rich in C7- to C9-aromatics, wherein
- the aromatics-rich starting hydrocarbon mixture is initially subjected to an extractive distillation in which the aromatics-containing hydrocarbon mixture is distilled with a suitable extracting solvent and freed therefrom to obtain a solvent-free product stream markedly depleted in aromatic hydrocarbons and a solvent-free aromatics concentrate markedly enriched in aromatic hydrocarbons,
**characterized in that**
- after the extractive distillation the solvent-free aromatics concentrate is passed into a reactor for hydrogenating dealkylation in which it is reacted with hydrogen to form a dealkylation aromatics stream as the main product and a paraffinic hydrocarbons stream and a methane stream as by-products and
- the aromatic hydrocarbon stream is passed into a treatment unit and
- the extractive distillation and the recovery of the extracting solvent are carried out in a single column.

2. Process according to Claim 1, **characterized in that** the treatment unit is a fractionating distillation.

3. Process according to Claim 1, **characterized in that** the treatment unit is a flash distillation stage.

4. Process for dealkylation of a hydrocarbon mixture rich in aromatics according to any of Claims 1 to 3, **characterized in that** the aromatics-containing starting product is fully hydrogenated pyrolysis gasoline.

5. Process for dealkylation of an aromatics-rich hydrocarbon mixture according to any of Claims 1 to 3, **characterized in that** the aromatics-containing starting product is catalytic reformate gasoline.

6. Process for dealkylation of aromatics-rich hydrocarbon mixture according to any of Claims 1 to 3, **characterized in that** the aromatics-containing starting product is pressure-refined coke oven benzene.

7. Process for dealkylation of an aromatics-rich hydrocarbon mixture according to any of Claims 1 to 6, **characterized in that** dealkylated and treated aromatics stream is pure benzene.

8. Process for dealkylation of an aromatics-rich hydrocarbon mixture according to any of Claims 1 to 7, **characterized in that** the aromatics-rich starting product is subjected to fractionating distillation in a distillation column before being sent into the extractive distillation.

9. Process for dealkylation of an aromatics-rich hydrocarbon mixture according to any of Claims 1 to 8, **characterized in that** before being sent into the extractive distillation the aromatics-rich starting product is brought into contact with water, with an acid, with a suitable solvent or with a mixture of these substances.

10. Process for dealkylation of an aromatics-rich hydrocarbon mixture according to any of Claims 1 to 9, **characterized in that** before being sent into the extractive distillation the aromatics-rich starting product is subjected to a hydrogenation.

11. Process for dealkylation of an aromatics-rich hydrocarbon mixture according to any of Claims 1 to 9, **characterized in that** a hydrogenation is carried out between the process step of the extractive distillation and the hydrogenating dealkylation.

12. Process for dealkylation of an aromatics-rich hydrocarbon mixture according to any of Claims 1 to 11, **characterized in that** the extracting solvent is an N-substituted solvent having 1 to 8 carbon atoms in the substituent.

13. Process for dealkylation of an aromatics-rich hydrocarbon mixture according to any of Claims 1 to 11, **characterized in that** the extracting solvent is N-formylmorpholine.

14. Process for dealkylation of an aromatics-rich hydrocarbon mixture according to any of Claims 1 to 11, **characterized in that** the extracting solvent for the extractive distillation is N-methylpyrrolidone, sulfolane, methylsulfolane, an alkylene glycol or an alkylated alkylene glycol or a mixture of these substances.

15. Process for dealkylation of an aromatics-rich hydrocarbon mixture according to any of Claims 12 to 14, **characterized in that** the extracting solvent mixture for extractive distillation contains water.

16. Process for dealkylation of an aromatics-rich hydrocarbon mixture according to any of Claims 1 to 15, **characterized in that** the hydrogenation step for hydrogenating dealkylation is a thermal process.

17. Process for dealkylation of an aromatics-rich hydrocarbon mixture according to any of Claims 1 to 15, **characterized in that** the hydrogenation step for hydrogenating dealkylation is a catalytic process.

18. Apparatus for dealkylation of an aromatics-rich hydrocarbon mixture comprising
- a rectifying column for the extractive distillation, a stripping column for removal of the extracting solvent, a reactor for hydrogenation of the produced aromatics concentrate, a purifying unit for treatment of the produced aromatics concentrate,
**characterized in that**
- the rectifying column, the stripping column, the hydrogenation reactor and the purifying unit are arranged such that an extractive distillation of aromatic hydrocarbons from a hydrocarbon-containing mixture, a removal of extracting solvent from the aromatic product stream, a hydrogenating dealkylation of aromatics to obtain a dealkylated aromatics concentrate and a treatment of the obtained aromatics concentrate are carried out successively in the process flow direction and
- the apparatus comprises a column which allows extractive distillation and recovery of the extracting solvent in a single column.

19. Apparatus for dealkylation of an aromatics-rich hydrocarbon mixture according to Claim 18, **characterized in that** it comprises a column which allows extractive distillation and recovery of the extracting solvent in a single column, wherein this column
- comprises a stripping part below the column main section, a rectifying part above the column main section, a column main section consisting of two parallel chambers and a column bottom with a dedicated bottom heating means and
- one chamber of the column main section is open to the column interior at the top and at the bottom and the other chamber is closed to the column interior at the top and open to the column interior at the bottom, wherein all chambers contain internals for improving mass transfer, and arranged above the internals of the chamber closed to the column interior at the top and open to the column interior at the bottom are means for vaporous withdrawal of an extractant-free product and for reflux of a liquefied product substream.

20. Apparatus for dealkylation of an aromatics-rich hydrocarbon mixture according to either of Claims 18 to 19, **characterized in that** it comprises a distillation unit for fractionating distillation arranged downstream of the unit for hydrogenating dealkylation in the process flow direction.

21. Apparatus for dealkylation of an aromatics-rich hydrocarbon mixture according to any of Claims 18 to 19, **characterized in that** it comprises a flash distillation stage arranged downstream of the unit for hydrogenating dealkylation in the process flow direction.

## Revendications

1. Procédé de désalkylation d'un mélange d'hydrocarbures riche en composés aromatiques en C₇ à C₉, selon lequel
- le mélange d'hydrocarbures de départ riche en composés aromatiques est tout d'abord soumis à une distillation extractive, lors de laquelle le mélange d'hydrocarbures contenant des composés aromatiques est distillé avec un solvant d'extraction approprié et débarrassé de celui-ci, un courant de produits fortement appauvri en hydrocarbures aromatiques et exempt de solvant et un concentré de composés aromatiques fortement enrichi en hydrocarbures aromatiques et exempt de solvant étant obtenus,
**caractérisé en ce que**
- le concentré de composés aromatiques exempt de solvant est acheminé après la distillation extractive dans un réacteur pour la désalkylation hydrogénante, dans lequel il est mis en réaction avec de l'hydrogène, un courant de composés aromatiques désalkylés étant formé en tant que produit principal et un courant d'hydrocarbures paraffiniques et un courant de méthane étant formés en tant que produits secondaires, et
- le courant d'hydrocarbures aromatiques est acheminé dans une unité de conditionnement, et
- la distillation extractive et la récupération du solvant d'extraction ont lieu dans une colonne unique.

2. Procédé selon la revendication 1, **caractérisé en ce que** l'unité de conditionnement consiste en une distillation fractionnée.

3. Procédé selon la revendication 1, **caractérisé en ce que** l'unité de conditionnement consiste en une étape de distillation « flash ».

4. Procédé de désalkylation d'un mélange d'hydrocarbures riche en composés aromatiques selon l'une quelconque des revendications 1 à 3, **caractérisé en ce que** le produit de départ contenant des composés aromatiques consiste en une essence de pyrolyse entièrement hydrogénée.

5. Procédé de désalkylation d'un mélange d'hydrocarbures riche en composés aromatiques selon l'une quelconque des revendications 1 à 3, **caractérisé en ce que** le produit de départ contenant des composés aromatiques consiste en une essence de reformat catalytique.

6. Procédé de désalkylation d'un mélange d'hydrocarbures riche en composés aromatiques selon l'une quelconque des revendications 1 à 3, **caractérisé en ce que** le produit de départ contenant des composés aromatiques consiste en un benzène de cokerie raffiné sous pression.

7. Procédé de désalkylation d'un mélange d'hydrocarbures riche en composés aromatiques selon l'une quelconque des revendications 1 à 6, **caractérisé en ce que** le courant de composés aromatiques désalkylés et conditionnés consiste en du benzène pur.

8. Procédé de désalkylation d'un mélange d'hydrocarbures riche en composés aromatiques selon l'une quelconque des revendications 1 à 7, **caractérisé en ce que** le produit de départ contenant des composés aromatiques est soumis à une distillation fractionnée dans une colonne de distillation avant l'introduction dans la distillation extractive.

9. Procédé de désalkylation d'un mélange d'hydrocarbures riche en composés aromatiques selon l'une quelconque des revendications 1 à 8, **caractérisé en ce que** le produit de départ contenant des composés aromatiques est mis en contact avec de l'eau, avec un acide, avec un solvant approprié ou avec un mélange de ces substances avant l'introduction dans la distillation extractive.

10. Procédé de désalkylation d'un mélange d'hydrocarbures riche en composés aromatiques selon l'une quelconque des revendications 1 à 9, **caractérisé en ce que** le produit de départ contenant des composés aromatiques est soumis à une hydrogénation avant l'introduction dans la distillation extractive.

11. Procédé de désalkylation d'un mélange d'hydrocarbures riche en composés aromatiques selon l'une quelconque des revendications 1 à 9, **caractérisé en ce qu'**une hydrogénation est réalisée entre l'étape de procédé de distillation extractive et la désalkylation hydrogénante.

12. Procédé de désalkylation d'un mélange d'hydrocarbures riche en composés aromatiques selon l'une quelconque des revendications 1 à 11, **caractérisé en ce que** le solvant d'extraction consiste en un solvant N-substitué contenant 1 à 8 atomes de carbone dans le substituant.

13. Procédé de désalkylation d'un mélange d'hydrocarbures riche en composés aromatiques selon l'une quelconque des revendications 1 à 11, **caractérisé en ce que** le solvant d'extraction consiste en la N-formylmorpholine.

14. Procédé de désalkylation d'un mélange d'hydrocarbures riche en composés aromatiques selon l'une quelconque des revendications 1 à 11, **caractérisé en ce que** le solvant d'extraction pour la distillation extractive consiste en la N-méthylpyrrolidone, le sulfolane, le méthylsulfolane, en un alkylène glycol ou en un alkylène glycol alkylé ou en un mélange de ces substances.

15. Procédé de désalkylation d'un mélange d'hydrocarbures riche en composés aromatiques selon l'une quelconque des revendications 12 à 14, **caractérisé en ce que** le mélange de solvants d'extraction pour la distillation extractive contient de l'eau.

16. Procédé de désalkylation d'un mélange d'hydrocarbures riche en composés aromatiques selon l'une quelconque des revendications 1 à 15, **caractérisé en ce que** l'étape d'hydrogénation pour la désalkylation hydrogénante consiste en un processus thermique.

17. Procédé de désalkylation d'un mélange d'hydrocarbures riche en composés aromatiques selon l'une quelconque des revendications 1 à 15, **caractérisé en ce que** l'étape d'hydrogénation pour la désalkylation hydrogénante consiste en un processus catalytique.

18. Dispositif pour la désalkylation d'un mélange d'hydrocarbures riche en composés aromatiques, comprenant :
- une colonne de rectification pour la distillation extractive, une colonne d'extraction pour l'élimination du solvant d'extraction, un réacteur pour l'hydrogénation du concentré de composés aromatiques formé, une unité de purification pour le conditionnement du concentré de composés aromatiques formé,
**caractérisé en ce que**
- la colonne de rectification, la colonne d'extraction, le réacteur d'hydrogénation et l'unité de purification sont agencés de telle sorte qu'une distillation extractive d'hydrocarbures aromatiques à partir d'un mélange contenant des hydrocarbures, une élimination du solvant d'extraction à partir du courant de produits aromatiques, une désalkylation hydrogénante de composés aromatiques pour obtenir un concentré de composés aromatiques désalkylés et un conditionnement du concentré de composés aromatiques obtenu aient lieu successivement dans le flux du procédé, et
- le dispositif comprend une colonne, qui permet une distillation extractive et la récupération du solvant d'extraction dans une colonne unique.

19. Dispositif pour la désalkylation d'un mélange d'hydrocarbures riche en composés aromatiques selon la revendication 18, **caractérisé en ce qu'**il comprend une colonne qui permet une distillation extractive et la récupération du solvant d'extraction dans une colonne unique, cette colonne
- comprenant une zone de rectification en dessous de la section principale de colonne, une zone d'enrichissement au-dessus de la section principale de colonne, une section principale de colonne constituée par deux chambres parallèles, et un fond de colonne auquel est attribué un chauffage de fond, et
- une chambre de la section principale de colonne étant ouverte vers le haut et vers le bas par rapport à l'espace intérieur de la colonne, et l'autre chambre étant fermée vers le haut par rapport à l'espace intérieur de la colonne et ouverte vers le bas par rapport à l'espace intérieur de la colonne, toutes les chambres contenant des composants intérieurs destinés à améliorer l'échange de matières, et des dispositifs pour le soutirage sous forme vapeur d'un produit exempt d'agent d'extraction et pour le reflux d'un courant partiel de produit liquéfié étant présents au-dessus des composants intérieurs de la chambre qui est fermée vers le haut par rapport à l'espace intérieur de la colonne et ouverte vers le bas par rapport à l'espace intérieur de la colonne.

20. Dispositif pour la désalkylation d'un mélange d'hydrocarbures riche en composés aromatiques selon l'une quelconque des revendications 18 à 19, **caractérisé en ce que** celui-ci comprend une unité de distillation pour la distillation fractionnée, qui est agencée après l'unité pour la désalkylation hydrogénante dans le flux du procédé.

21. Dispositif pour la désalkylation d'un mélange d'hydrocarbures riche en composés aromatiques selon l'une quelconque des revendications 18 à 19, **caractérisé en ce que** celui-ci comprend une étape de distillation « flash », qui est agencée après l'unité pour la désalkylation hydrogénante dans le flux du procédé.
